# EUROPEAN PATENT APPLICATION

(11) **EP 1 216 701 A1**
(43) Date of publication of application: **26.06.2002**
(21) Application number: 00250456.1
(22) Date of filing: 22.12.2000
(51) Int. Cl.: A61K 31/00, A61K 31/57, A61K 31/5685, A61K 31/568, A61K 31/575, A61K 31/573, A61K 31/565, A61K 31/569, A61K 31/277, A61K 31/138, A61K 31/428, A61P 15/08, A61P 15/16, A61P 15/18

(54) **Process to increase the concentration of meiosis-activating sterols (MAS) in the cholesterol synthesis and the use of potent inhibitors in the process**

(71) Applicant: Schering Aktiengesellschaft, 13353 Berlin (DE)
(72) Inventor: Lindenthal, Bernhard, Dr., 53127 Bonn (DE)

(57) **Abstract**

The invention relates to a process to increase the concentration of meiosis-activating sterols (MAS) in the cholesterol synthesis wherein potent inhibitors where added, the use of potent inhibitors in the cholesterol synthesis, a pharmaceutical composition comprising the potent inhibitors and the use of the inhibitors for the microbiology production of meiosis-activating sterols (MAS). In the process of the invention the Δ24-reduction or/and 4α-demethylation in the cholesterol synthesis are inhibited. As a result of this inhibition the concentration of FF-MAS and/or T-MAS as meiosis-activating sterols (MAS) are increased.

## Description

The present invention relates to a process to increase the concentration of meiosis-activating sterols (MAS) in the cholesterol synthesis wherein potent inhibitors where added, the use of potent inhibitors in the cholesterol synthesis, a pharmaceutical composition comprising the potent inhibitors and the use of the inhibitors for the microbiology production of meiosis-activating sterols (MAS).

In the process of the invention the Δ24-reduction or/and 4α-demethylation in the cholesterol synthesis are inhibited. As a result of this inhibition the concentration of FF-MAS and/or T-MAS as meiosis-activating sterols (MAS) are increased.

Very potent inhibitors are Progesterone, Pregnenolone, 17α-Hydroxy-pregnenolone, 17α-Hydroxy-progesterone, 4-Androsten-3,17-dione, Testosterone, Medroxyprogesterone, Verapamil, Tamoxifen, Ursodeoxycholic acid (UDCA), Chenodeoxycholic acid (CDCA), Deoxycholic acid (DCA), Lithocholic acid (LCA), Cortisone, Cortisol, 11-Desoxycortisol, 17β-Estradiol, Aldosteron, Dehydroepiandrosteron, Norethynodrel, 11-Deoxycorticosterone, Corticosterone, 6-Amino-2-n-pentylthiobenzothiazole, or mixtures of them.

This invention disclose also the use of the potent inhibitors in the cholesterol synthesis to increase the concentration of meiosis-activating sterols (MAS), the use for the manufacture of a medicament for the control of fertility possibly in a mixture with pharmaceutically acceptable auxiliaries and the use for the microbiology production of meiosis-activating sterols (MAS).

### Background of the Invention

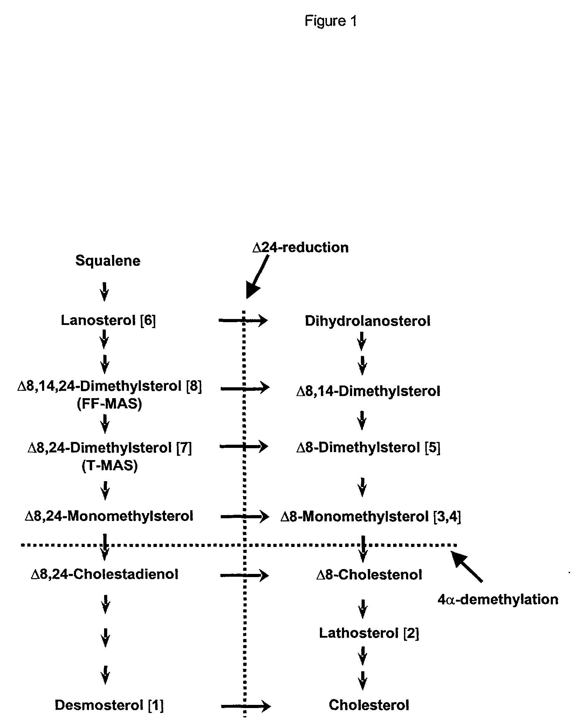

The biosynthetic pathway of cholesterol comprises the conversion of lanosterol to cholesterol (Figure 1). A large number of intermediate sterols are involved to complete cholesterol synthesis from lanosterol, the first sterol precursor of cholesterol. Some intermediate cholesterol precursors can be measured in human serum and have been shown useful indicators of cholesterol synthesis. Their serum concentrations are influenced by 1) physiological conditions 2) various diseases and 3) drug treatments. Furthermore, various agents are known to affect specific steps of cholesterol synthesis and recent reports showed that Amphotericin and AY 9944-A-7 are able to affect late steps of cholesterol synthesis (WO 00/52142).

The classic pathway from mevalonate to cholesterol has several ancillary functions. Isoprenoid intermediates are partially diverted to protein prenylation, isopentenylation of tRNA, and production of dolichols and ubiquinone. Small amounts of 7-dehydrocholesterol are converted to vitamin D₃, and other intermediates are oxidized to regulatory species. Although the actual intermediates of cholesterol biosynthesis might appear to have no special biochemical or physiological functions, recent evidence (1) suggests that C₂₉ precursors of cholesterol are the long-sought (2,3) meiosis-inducing substances. Two notable examples of C₂₉ meiosis-activating sterols (MAS) are FF-MAS (4,4-dimethyl-5a-cholesta-8,14,24-trien-3β-ol), isolated from human follicular fluid, and T-MAS (4,4-dimethyl-5α-cholesta-8,24-dien-3β-ol), isolated from bull testis (1). Synthetic FF-MAS (1,4,5) and other C₂₉ sterols (1,5) overcome meiotic arrest produced by hypoxanthine, dbcAMP or 3-isobutyl-1-methylxanthine in naked oocytes or cumulus enclosed oocytes. In contrast, lanosterol, cholesterol, Δ5,7- and Δ5-C₂₉ sterols, and several oxysterols fail to activate meiosis (4-7). The proposed regulation of meiosis by C₂₉ sterols is supported by the finding that MAS are synthesized in rat and human gonads (8,9), but the mechanism by which sterols activate meiosis is unclear (10,11). Mediation by an LXR receptor, suggested (6) on the basis of results that some meiosis-activating sterols are also ligands for the LXRα receptor (5-7,12), has been discounted (12).

Another unsolved problem, addressed herein, is the mechanism by which levels of MAS are regulated. Under normal metabolic conditions, the rapid conversion of sterol intermediates to cholesterol results in low concentrations of precursors in liver, blood and most other tissues. Exceptions include the occurrence of elevated concentrations of T-MAS in testicular tissue (1,11), of FF-MAS and T-MAS in preovulatory follicular fluid (11) and of desmosterol in spermatozoa, testis (13,14), human milk (15), and the developing brain (16). The significant levels of MAS in gonadal tissue could result from hormonal regulation, and potent inhibitors have long been associated with meiotic events (17,18) and with inhibition of cholesterol synthesis in somatic cells (19,20). Extensive results in a variety of cell lines indicate that progesterone inhibits sterol synthesis and that the progesterone receptor is not involved (21-23). Lanosterol and several other sterol precursors accumulated but were not identified or characterized beyond their TLC or HPLC mobility.

### Summary of the Invention

According to the present invention there are potent inhibitors in the cholesterol biosynthesis which are added and the concentrations of meiosis-activating sterols (MAS) will increase.

The term meiosis-activating sterols (MAS) refers to a substance which is an intermediate in the cholesterol biosynthesis between lanosterol and cholesterol (Fig. 1). Two examples of MAS are FF-MAS (4,4-dimethyl-5α-cholesta-8,14,24-triene-3β-ol) and T-MAS (4,4-dimethyl-5α-cholest-8,24-diene-3β-ol). A MAS will preferably be a sterol. Examples of MAS compounds are mentioned in WO 96/00235, WO 96/27658, WO 97/00883, WO 97/00884, WO 98/28323, WO 98/54965, WO 98/55498 and WO 00/35938. It is preferred that the MAS is selected from the group consisting of FF-MAS, T-MAS, 1-methyl-zymosterol and zymosterol.

The term potent inhibitors refers to substances which interfere with the cholesterol biosynthesis in a way which results in accumulation (increasing) of meiosis activating sterols.

The invention discloses for example Progesterone, Pregnesolone, Pregnenolone, 17α-Hydroxy-pregnenolone, 17α-Hydroxy-progesterone, Androsten-3,17-dione, Testosterone, Medroxyprogesterone, Verapamil, Tamoxifen, Ursodeoxycholic acid, Chenodesoxycholic acid, Desoxycholic acid, Lithocholic acid, Cortisone, Cortisol, 11-Desoxycortisole, 17β-Estradiole, Aldosterone, Dehydroepiandrosteron, Norethynodrel, 11-Deoxycorticosterone, Corticosterone, 6-Amino-2-n-pentylthiobenzothiazole or mixtures of them as potent inhibitors in the cholesterol synthesis.

These potent inhibitors can be used in vivo and in vitro to inhibit the cholesterol biosynthesis and increase the meiosis-activating sterols. The meiosis activating sterols are responsible for the control of fertility, for example additives or additives capable of endogenous stimulation of the accumulation for in vitro fertilization, the pre-embryo resulting from the in vitro fertilization having an improved implantation rate in vivo.

One aspect of the inventions relates to the potent inhibitors which increase the ovarian follicular cumulus cell production of MAS and thereby enhance the capacity of the oocyte to undergo normal pre-embryo development resulting in augmented implantation and conceptional potential of the derived pre-embryos obtained during a culture period as used in connection with assisted reproduction and infertility treatment.

The invention relates also to the use of potent inhibitors in the cholesterol synthesis to increase the concentration of meiosis-activating sterols (MAS), the use for the manufacture of a medicament for the control of fertility and the use for the microbiology production of meiosis-activating sterols (MAS), in a given teaching by M. Bard et al., [Proc. Natl. Acad. Sci. (USA) (1996), 93, 186 - 190.]

The potent inhibitors also referred to herein as the active ingredient, may be administered enterally or parenterally. The exact dose and regimen of administration of the active ingredient, or a pharmaceutical composition thereof, will necessarily be dependent upon the therapeutic effect to be achieved (contraception or infertility), and will vary with the particular compound, the route of administration, and the age and condition of the individual subject to whom the medicament is to be administered.

In general, parenteral administration requires lower dosages than other methods of administration which are more dependent upon adsorption. However a dosage for humans preferably contains 0.0001- 250 mg per kg body weight. The desired dose may be presented as one dose or as multiple subdoses administered at appropriate intervals throughout the day, or, in case of female recipients, as doses to be administered at appropriate daily intervals throughout he menstrual cycle. The dosage as well as the regimen of administration may differ between a females and a male recipient.

In case of in vitro or ex vivo applications, the compounds of the invention are to be used in the incubation media in a concentration of approximately 0.01 - 500 µg/ml.

### Detailed disclosure of the Invention

In a major extension of these (21-23,24) and earlier (19) findings, we now report incubations of HepG2 cells and rat testis with potent inhibitors at concentrations commonly found in gonadal tissue. Our results in HepG2 cells confirm the reported (22-24) inhibition of cholesterol synthesis, the accumulation of cholesterol precursors, and the lack of involvement of the progesterone receptor. Moreover, we have demonstrated by GC/MS that major accumulating sterols are identical with authentic standards of T-MAS and FF-MAS. This critical structural information indicates a previously unrecognized linkage between potent inhibitors and MAS. Based on our full experimental results, we propose a mechanism leading to the accumulation of MAS through the inhibition of two steps in the cholesterol synthesis pathway (Fig. 1) by specific individual potent inhibitors. Our combined results provide the first credible explanation for the presence of micromolar levels of MAS in gonadal tissue and bring together previously unconnected lines of research, i.e. the biochemical role of MAS in fertility and the effects of potent inhibitors on cholesterol biosynthesis and on meiosis.

The resumption of meiosis is regulated by meiosis-preventing and meiosis-activating substances in testes and ovaries. Certain C₂₉ precursors of cholesterol are present at elevated levels in gonadal tissue, but the mechanism by which these meiosis-activating sterols (MAS) accumulate has remained an unresolved question. Here we have found that potent inhibitors alter cholesterol synthesis in HepG2 cells and rat testes to increase levels of major MAS (FF-MAS and T-MAS). These C₂₉ sterols accumulated as a result of inhibition of Δ24-reduction and 4α-demethylation. Progesterone, pregnenolone and 17α-OH-pregnenolone were potent inhibitors of Δ24-reduction in an *in vitro* cell assay and led to the accumulation of desmosterol, a Δ5,24 sterol precursor of cholesterol. A markedly different effect was observed for 17α-OH-progesterone, which caused the accumulation of sterols associated with inhibition of 4α-demethylation. The flux of ¹³C-acetate into lathosterol and cholesterol was decreased by potent inhibitors as measured by isotopomer spectral analysis, whereas newly synthesized MAS accumulated. The combined evidence that MAS concentrations can be regulated by physiological levels of potent inhibitors and their specific combination provides a plausible explanation for the elevated concentration of MAS in gonads and suggests a new role for potent inhibitors in fertility.

### RESULTS

### Accumulation of cholesterol precursors in HepG2 cells treated with potent inhibitors

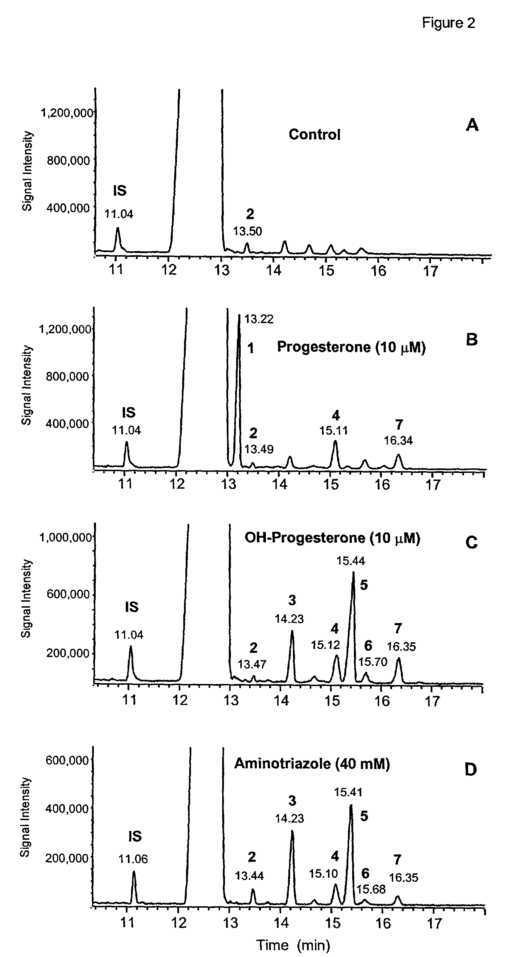

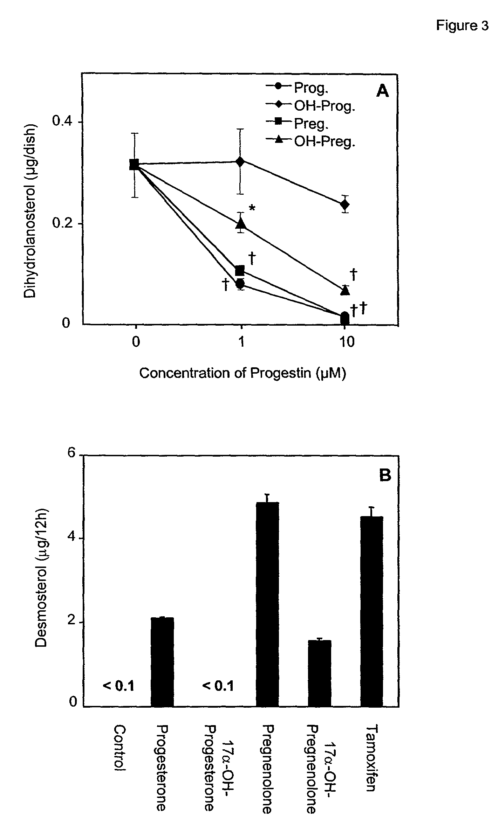

Incubation of HepG2 cells with progesterone, pregnenolone or 17α-OH-pregnenolone led to the accumulation of desmosterol, a cholesterol precursor in which the Δ24 bond has not been reduced (Fig. 2B and Fig. 3B). In contrast 17α-OH-progesterone produced an accumulation of methyl and dimethyl sterols (Fig. 2C), including T-MAS and 4,4-dimethyl-5α-cholest-8-en-3β-ol, a meiosis-activating sterol (1) that was identified by comparison with MS data reported by Hashimoto et al. (27). These effects resemble the action of aminotriazole (Fig. 2D), which inhibits 4α-demethylation (27) and other heme-dependent processes that may affect sterol metabolism in peroxisomes and endoplasmic reticulum. An inhibition of Sterol 4-Demethylation by 6-Amino-2-n-Pentylthiobenzothiazol is also reported in the literature (46 and 47). These accumulation patterns are consistent with the inhibition of Δ24-reduction (by progesterone at 10 µM) and 4α-demethylation (by 17α-OH-progesterone at 10 µM and aminotriazole at 40 mM). Compared to potent inhibitors, other hormones (testosterone, β-estradiol, estrone, dehydroepiandrosterone and androstenedione; at 10 µM) produced only minor changes in the cholesterol precursor pattern (data not shown). Despite an accumulation of desmosterol after a 12-h incubation with 10 µM progesterone, desmosterol amounted to only 2.1% of the cholesterol content of the cells (5.7 ± 0.07 µg desmosterol vs. 269 ± 3 µg cholesterol, n=3 incubations).

### Effect of potent inhibitors on Δ24-reduction and desmosterol accumulation

To isolate Δ24-reduction from other steps of the pathway, the conversion of lanosterol to dihydrolanosterol was studied in HepG2 cells by blocking further metabolism with added miconazole (Fig. 3A). Incubations with various potent inhibitors showed the following order of suppression of Δ24 reduction: pregnenolone > progesterone > 17α-OH-pregnenolone >> 17α-OH-progesterone. In the absence of miconazole, the Δ24 reduction step was strongly suppressed by the antiestrogen drug tamoxifen and by pregnenolone, progesterone and 17α-OH-pregnenolone, but not by 17α-OH-progesterone (Fig. 3B). The observed accumulation of desmosterol in HepG2 cells in the absence of miconazole (Fig. 3B) indicates that all steps of cholesterol biosynthesis are intact except the Δ24 reduction.

### Identification of T-MAS and FF-MAS

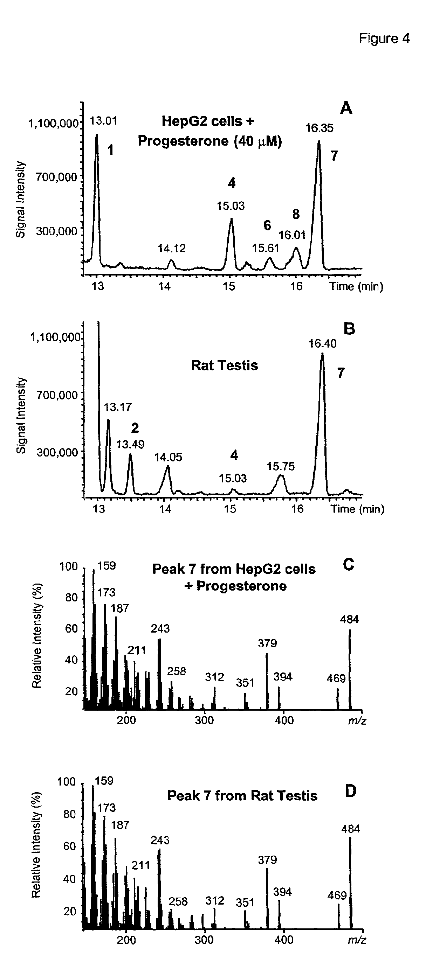

Treatment of HepG2 cells with a higher concentration of progesterone (40 µM) produced increased levels of methylsterols (compounds 4 and 7, Fig. 4A) in addition to desmosterol (compound 1, Fig. 4A). The dominant cholesterol precursor was a diunsaturated C₂₉ sterol also found in adult rat testis (Fig. 4B). These sterols were both identified as T-MAS (4,4-dimethyl-5α-cholesta-8,24-dien-3β-ol) by comparison of their GC retention times and mass spectra (Fig. 4C and D) with those of a synthetic standard (5) for T-MAS. FF-MAS (4,4-dimethyl-5α-cholesta-8,14,24-trien-3β-ol) (1), was also detected in the HepG2 cells (Fig. 4A, compound 8) and testis extracts and identified by comparison of its GC retention time and mass spectrum with those of the reference compound (5). The identities of T-MAS and FF-MAS were further confirmed by analysis of testis extracts spiked with various amounts of authentic standards of MAS. The observed pattern of cholesterol synthesis intermediates was consistent with inhibition of both 4α-demethylation and Δ24-reduction.

### Synergistic and additive effects of potent inhibitors and drugs

Incubation of HepG2 cells and rat testis slices with individual potent inhibitors and drugs altered the levels of the three C₂₉-sterols (Table 1). In many cases, combinations of 17α-OH-progesterone with other potent inhibitors produced additive and/or synergistic effects (Table 1). These effects were less pronounced in testis slices, possibly as a consequence of a lower total cholesterol synthesis rate relative to that of the highly metabolic HepG2 cells, the higher background of pre-existing MAS in testes and/or the shorter incubation time. As shown in Table 1, the effects of potent inhibitors, both alone and in combination, were similar to those caused by aminotriazole, a known inhibitor of 4α-demethylation (27), tamoxifen, an inhibitor of Δ24-reduction and Δ7-Δ8 isomerization (26), and verapamil, a calcium blocker used in treating cardiovascular diseases. Medroxyprogesterone, a synthetic progestin used as a contraceptive, also led to large increases in C₂₉-sterols. A combination of β-estradiol (10 µM), the second major hormone in follicular fluid, and progesterone (10 µM) did not alter the accumulation of cholesterol precursors observed with progesterone alone.

### T-MAS levels in the developing rat testis

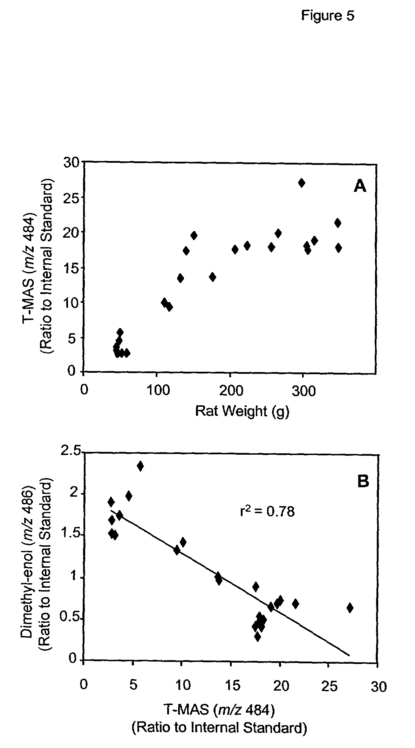

To detect changes in cholesterol precursors in developing rat testis, neutral sterols were measured by GC-MS in young rats (body weight < 60 g) and adult rats (body weight 100-350 g). The results (Fig. 5A) show that T-MAS levels were ca. 4 times higher in the adult rats than in young rats and that T-MAS becomes the dominant cholesterol precursor (Fig. 4B). No other cholesterol precursor showed such a pronounced change during maturation. In fact cholesterol precursors later in the biosynthetic pathway (e.g. lathosterol and Δ8-cholestenol) were reduced by ca. 50% in adult rats. Interestingly, the relationship between 4,4-dimethyl-5α-cholest-8-en-3β-ol (no Δ24 bond) and T-MAS (Δ24 double bond present) exhibited a strong inverse correlation, as shown in Fig. 5B. This suggests that the shift of cholesterol precursors in the developing rat testis involves impaired Δ24-reduction.

### Influence of potent inhibitors on flux of ¹³C acetate to lathosterol and cholesterol

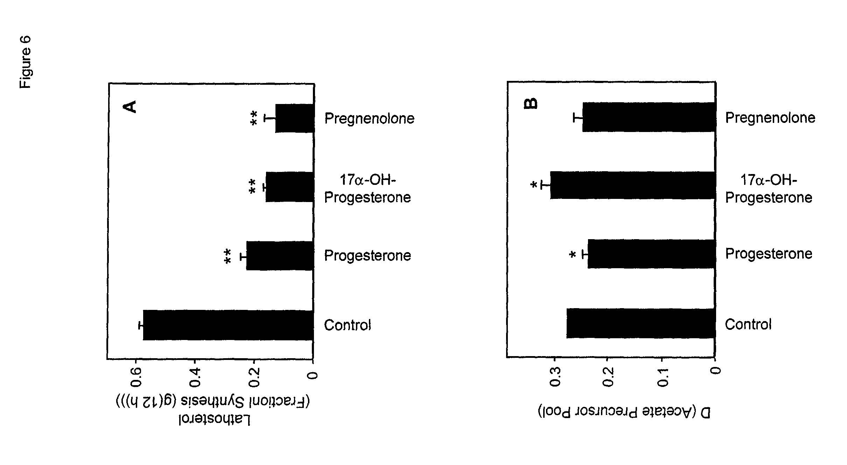

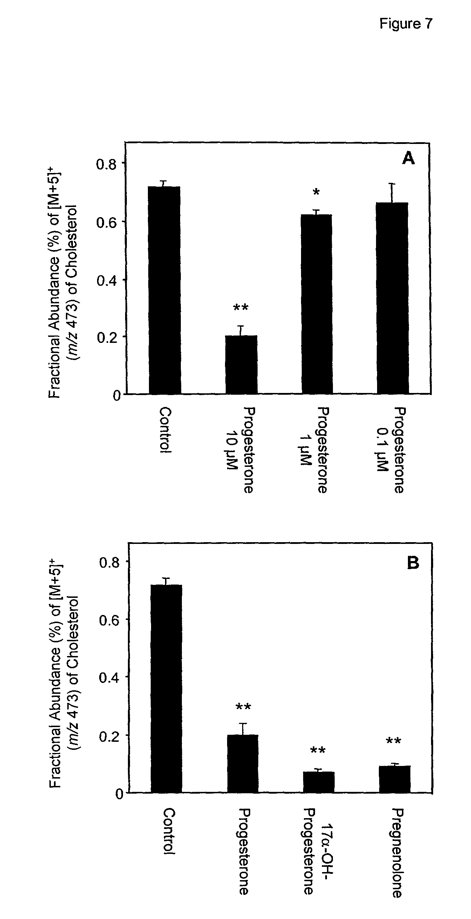

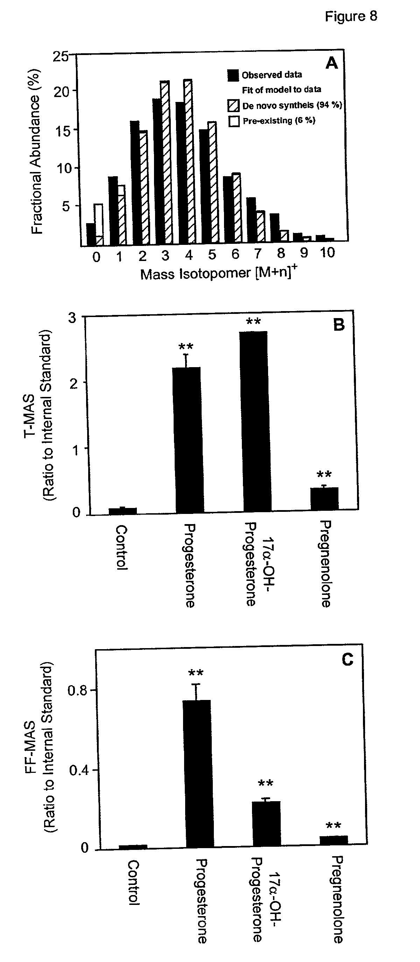

To investigate possible mechanisms of action of the potent inhibitors, we used ISA to determine the flux of ¹³C acetate to cholesterol precursors later in cholesterol synthesis. We measured the precursor pool enrichment D and the fractional synthesis rate g(t) of lathosterol (5α-cholest-7-en-3β-ol) in HepG2 cells in the presence of various potent inhibitors (Fig. 6). Progesterone, 17α-OH-progesterone and pregnenolone reduced de novo lathosterol synthesis by more than 60%, and the total amount of lathosterol was lowered by ca. 50%. De novo fractional cholesterol synthesis g(t) was 4.2% ± 0.2 (n=3) for controls but not detectable under the influence of potent inhibitors. Reduced flux of ¹³C-acetate to cholesterol was also indicated by the markedly lower fractional abundance of the ¹³C isotopomers ([M+3]⁺ to [M+8]⁺) of cholesterol in the treated cells (Fig. 7). Despite the very small MS responses, we observed a reduced fractional abundance for [M+5]⁺ in progestin-treated cells (Fig. 7B), with a dose-dependent lowering for progesterone (Fig. 7A). The combined results show that potent inhibitors block de novo cholesterol synthesis at steps before lathosterol. In contrast to lathosterol, ¹³C-T-MAS did accumulate (Fig. 8), indicating that de novo synthesis of intermediates prior to Δ24-reduction and 4α-demethylation was not affected by the potent inhibitors.
All the added potent inhibitors produced an acetate precursor pool enrichment D (proportion of ¹³C-acetate in the acetate pool for sterol synthesis) of 0.2-0.3 (Fig. 6B), with a slight but statistically significant difference for progesterone and 17α-OH-progesterone. This difference might arise from differing concentrations of cholesterol precursors, which, through feedback inhibition of HMG-CoA reductase, may ultimately affect the acetate precursor pool. If so, investigation of effects of inhibitors of biosynthetic pathways on precursor pools might offer a useful tool for understanding metabolic regulation.

### Accumulation of ¹³C labeled T-MAS and FF-MAS in progestin-treated HepG2 cells

To test whether the accumulating T-MAS and FF-MAS are derived from newly synthesized sterols, we incubated HepG2 cells with potent inhibitors (10 µM) and 1-¹³C-acetate (1 mM) for 12 h. The isotopomer distribution of T-MAS after incubation with 17α-OH-progesterone showed a preponderance of ¹³C-containing species. The fit of the T-MAS isotopomer distribution to the ISA model indicated that T-MAS is derived primarily from new synthesis (Fig. 8A). The effect of various potent inhibitors on the accumulation of T-MAS and FF-MAS is shown in Fig. 8B and C. These results show that highly labeled and therefore newly synthesized MAS accumulated during incubation with progesterone and 17α-OH-progesterone.

### The progesterone receptor is not involved in blocking cholesterol synthesis

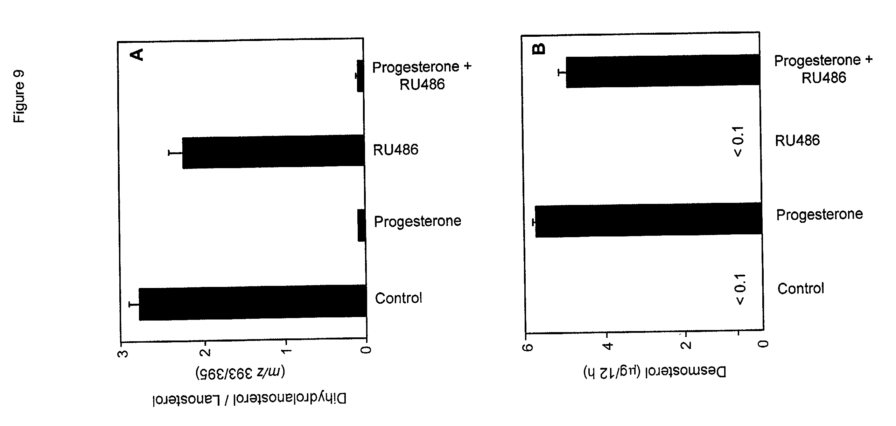

To investigate the possible involvement of the progesterone receptor in the actions of progesterone, we incubated HepG2 cells with the progesterone antagonist RU486 (2 µM) alone or in the presence of progesterone (10 µM). As shown in Fig. 9A, RU486 did not counteract the dramatic decline in the ratio of dihydrolanosterol to lanosterol nor the accumulation of desmosterol observed upon incubation with progesterone (Fig. 9B).

### DISCUSSION

This invention demonstrates that treatment of a hepatic cell model with potent inhibitors alters cholesterol synthesis and leads to the accumulation of MAS. In HepG2 cells at 10 µM concentration, progesterone, pregnenolone and 17α-OH-pregnenolone produced a pattern of precursors consistent with inhibition of Δ24-reduction (Fig. 2B and Fig. 3). In contrast, 17α-OH-progesterone produced a different pattern (notably methyl and dimethyl sterols) corresponding to inhibition of 4α-demethylation (Fig. 2C). Incubations of potent inhibitors in the presence of miconazole (Fig. 3A), which blocks 14α-demethylation, demonstrated that the effects of the potent inhibitors on Δ24-reduction are not mediated through other cholesterol precursors that may accumulate in cells when the entire pathway is intact. 17α-OH-Progesterone led to the accumulation of the same dimethyl and methyl cholesterol precursors as aminotriazole (Fig. 2C and D), a known (albeit less potent) inhibitor of 4α-demethylation (27). Our findings represent the first report of the effects of 17α-OH-progesterone on cholesterol synthesis. These effects are distinct from the actions of other potent inhibitors described herein. Incubations with potent inhibitors in the presence of ¹³C-acetate led to the accumulation of ¹³C-MAS (Fig. 8). These results demonstrate that the MAS arose from de novo synthesis rather than interconversion of pre-existing sterols. Potent inhibitors were also capable of increasing the levels of MAS in testicular slices (Table 1).
We found that simultaneous inhibition of Δ24-reduction and 4α-demethylation by potent inhibitors leads to the accumulation of MAS. Additive and synergistic effects of 17α-OH-progesterone with either pregnenolone or progesterone on the levels of T-MAS and FF-MAS (Table 1) are compatible with this hypothesis. Furthermore, we found that progesterone, pregnenolone and 17α-OH-progesterone in HepG2 cells (Fig. 6 and 7) and rat testis slices suppressed the incorporation of ¹³C-acetate into sterols requiring both Δ24-reduction and 4α-demethylation (e.g. lathosterol and cholesterol) (Fig. 6 and 7) and led to the accumulation of labeled MAS (Fig. 8). It is also notable that the dominant cholesterol precursor in rat testis is T-MAS (Fig. 4B) and that T-MAS accumulates in the developing rat testis (Fig. 5A) while levels of 4,4-dimethyl-5α-cholest-8-en-3β-ol decline during maturation (Fig. 5B). These observations support our hypothesis that Δ24-reductase activity is suppressed in the adult rat testis.
The effects described herein of potent inhibitors on cholesterol synthesis occurred at normal physiological concentrations for gonadal tissue. Increases in T-MAS (9 fold) and FF-MAS (4 fold) occurred in the presence of 1 µM progesterone or 1 µM 17α-OH-progesterone (Table 1). These concentrations are comparable to reported levels in human testes (505 ng/g (ca. 1.6 µM) pregnenolone; (306 ng/g (ca. 0.9 µM) 17α-hydroxyprogesterone; and 81 ng/g (ca. 0.3 µM) progesterone (wet weight)) (28) and human follicular fluid (8-25 µM progesterone; 3-6 µM 17α-OH-progesterone) (29). In evaluating the ramifications of progestin levels, it should be noted that a permanent induction of meiosis is required in men, whereas the resumption of meiosis in women coincides with the progestin surge induced by lutenizing hormone (30). We also found that the effects of progesterone on the accumulation of desmosterol and on the ratio of dihydrolanosterol to lanosterol were not modulated by the progesterone receptor blocker RU486 (2 µM) (Fig. 8). This result and our other observations are in agreement with previous reports (19,21-23,32) that steroid hormones affect cholesterol synthesis in various cell types, leading to an accumulation of cholesterol precursors, and that the progesterone receptor is not involved.

In seeking an explanation for the effects of progesterone on cholesterol synthesis, some authors have investigated mechanisms based on the interference of progesterone with intracellular cholesterol trafficking (23,31-35). Our results suggest an additional or alternative mechanism involving direct inhibition of specific steps of sterol synthesis. In efforts to understand the synthetic origins of MAS, two groups have reported upregulation of sterol 14α-demethylase (P450_{14DM}) (8,9). Such an increase in the activity of the 14α-demethylase would likely result in simultaneous accumulation of dimethylsterols with and without a Δ24 bond, whereas we observed an actual decrease in levels of monounsaturated dimethylsterols and a concomitant increase in levels of T-MAS in developing rat testes (Fig. 5B). Our results indicate impaired Δ24 reduction rather than enhanced activity of the 14α-demethylase. The observed pattern of precursors is compatible with a "downstream" mechanism of MAS regulation consisting of inhibition of later steps in the cholesterol biosynthetic pathway to divert flux into MAS. Under normal circumstances this pathway operates such that cholesterol precursors do not accumulate. Interestingly, progesterone (10 µM) has been shown to increase the activity of HMG-CoA reductase in human fibroblasts (33). This observation is consistent with decreased feedback inhibition of cholesterol (or C₂₇-oxysterols) on HMG-CoA reductase, effects that would be anticipated from our finding that added progesterone decreases flux from ¹³C-acetate to lathosterol.
Although the suggested connections between potent inhibitors, MAS, and fertility represent an attractive explanation that unifies a considerable body of observations, our results do not exclude alternative scenarios. For example, low micromolar MAS accumulation produced by potent inhibitors might be physiologically irrelevant to the regulation of meiosis. Related to this caveat is our inability to fully explain the accumulation of FF-MAS in follicular fluid and ovarian tissue, a process that may involve inhibition of Δ14-reductase. Although hepatocytes contain enzymes not expressed in gonadal tissue, HepG2 cells are otherwise a useful experimental model because of their high rate of cholesterol synthesis in lipid-deficient media and their low background of pre-existing MAS. It should also be noted that, although our GC/MS evidence for FF-MAS and T-MAS exceeds commonly accepted standards for sterol identification, reported limitations of GC/MS (36) point to the value of more definitive analyses in future work, which should also include measurements of MAS levels relative to cell protein.
The mechanism of action suggested by our results may bear directly on the role of potent inhibitors in human fertility. The importance of changing from an estrogen/androgen secreting status to a predominately progesterone secreting status in human ovaries has been emphasized by several investigators (29,37-39) and coincides with the resumption of meiosis (30,40). Impairment of progesterone secretion is associated with atretic follicles, and low progesterone levels have been reported in the empty follicle syndrome (41,42). In some oligospermic men, abnormal steroidogenesis with lower levels of testicular 17α-OH-progesterone has been reported (43). These requirements of potent inhibitors in normal fertility may be explained by our findings that potent inhibitors stimulate the production of sterols known to activate meiosis (1,5,12). Increases in desmosterol levels in primate testis parallel the onset of changes in testosterone synthesis (14), and activation of meiosis is associated with the onset of spermatogenesis (44). Evidence for the synthesis of MAS in rat gonads has recently been presented by Yoshida et al. (8). Our data further suggest that elevated progestin levels, leading to inhibition of Δ24-reduction, provide a coherent explanation for the relatively high concentrations of desmosterol in semen, testes (13,14), mothers' serum before and after delivery (45), and human milk (15).
In summary, we provide evidence that potent inhibitors may be natural regulators of MAS. Our findings point to a new role for potent inhibitors in fertility through synergistic actions of 17α-OH-progesterone with other potent inhibitors and suggest potential targets for possible pharmacological intervention. However, the relevance of our experimental results to the regulation of meiosis presupposes a physiological role for MAS in fertility, a matter that remains to be demonstrated conclusively.

Thus, when the potent inhibitors of this invention are to be administered to a mammal, they are conveniently provided in the form of a pharmaceutical composition which comprises at least one compound of the potent inhibitors in connection with a pharmaceutically acceptable carrier. For oral use, such compositions are preferably in the form of capsules or tablets.

From the above it will be understood that administrative regimen called for will depend on the condition to be treated. Thus, when used in the treatment of infertility the administration may be once only, or for a limited period, e.g. until pregnancy is achieved. When used as a contraceptive, the meiosis inducing substance with either have to be taken continuously or cyclically. when used as a contraceptive by women and not taken continuously, the timing of the administration relative to the menstrual cycle will be important.

The pharmaceutical compositions may comprise carriers, diluents, absorption enhancers, buffers, agents for adjusting the osmotic pressure, tablet disintegrating agents and other ingredients which are conventionally used in the art. Examples of solid carriers are magnesium carbonate, magnesium stearate, dextrin, lactose, sugar, talc, gelatin, pectin, tragacanth, methyl cellulose, sodium carboxymethyl cellulose, low melting waxes and cocoa butter.

Liquid compositions include sterile solutions, suspensions and emulsions. Such liquid compositions may be suitable for injection or for use in connection with ex vivo and in vitro fertilization. The liquid compositions may contain other ingredients which are conventionally used in the art.

Further, a composition for transdermal administration of a compound of this invention may be provided in the form of a patch and a composition for nasal administration may be provided in the form of a nasal spray in liquid or powder form.

### EXAMPLES

### MATERIALS AND METHODS

### Materials

Unless specified otherwise, potent inhibitors, drugs, chemicals, and cell culture supplies were purchased from Sigma. T-MAS and FF-MAS were synthesized as reported recently (5) and used as reference compounds. 1-¹³C-acetate was obtained from Cambridge Isotope Laboratories. Rat testes were obtained from Sprague-Dawley rats (weight <50g).

### Cell culture

Human hepatoma HepG2 cells were cultured to confluency in 60-mm dishes in phenol-red-free Dulbecco's modified Eagle's medium (DMEM) containing Pen-Strep (100 µg/ml), glucose (10 mM), sodium bicarbonate (45 mM) and glutamine (4 mM) supplemented with 10% fetal calf serum as described previously (24). At the beginning of the experiment the medium was changed to serum-free DMEM supplemented with 10% lipid-free "controlled process serum replacement" (CPSR-1). Compounds were added from ethanolic stock solutions to produce final ethanol concentrations of 0.1-0.3% v/v. Controls received the same amount of ethanol. Aminotriazole was added in an aqueous solution. Slices of rat testis (25 µm; 25-50 mg wet weight) were incubated under the same conditions in a shaking water bath (37°C). For isotopomer spectral analysis (ISA) of lathosterol, 1-¹³C-acetate was added in an aqueous solution to a final concentration of 1 mM.

### Sterol extraction and analysis

Cells were washed three times with ice cold PBS (pH 7.4) (once with 2 mg/ml BSA and twice with PBS alone). Epicoprostanol (1 µg in 50 µl of hexane) was added as internal standard to each sample. Sterols were extracted in hexane/isopropanol (3:2 v/v; two times with 4 ml). After drying under nitrogen and alkaline hydrolysis (1 N NaOH in 80% ethanol, 1.5 h at 70°C), the sterols were extracted with cyclohexane (two times with 3 ml). After drying under nitrogen, sterols were converted to their trimethylsilyl (TMS) derivatives by adding 100 µl bis(trimethylsilyl)trifluoracetamide (Pierce)/n-decane (1:1; v/v) and heating for 1 h at 70°C in a conical glass tube. This solution (1-2 µl) was used for gas-chromatography/mass-spectrometry (GC/MS). Rat testes (0.05-0.15 g) were homogenized manually, and lipids were extracted in hexane/isopropanol (3:2 v/v; two times with 4 ml). The extracts were deproteinized with acetone (3 ml) and processed as described above. The observed sterol levels in testis were normalized to correspond to 0.1 g of tissue (wet weight).

### Gas-chromatographylmass-spectrometry

Analyses were performed on a Hewlett-Packard GC/MS system (5890 series II GC interfaced to a 5971 mass selective detector) equipped with a DB-XLB column (30 m x 0.25 mm i.d. x 0.25 µm film; J&W). Gas-chromatography was done in the splitless mode with temperature programming as follows: 150°C for 1 min, followed by 20°C/min up to 260°C and 10°C/min up to 280°C (hold for 15 min). Mass spectral data were collected either in the full-scan mode (*m*/*z* 50-550) or by selective ion monitoring (SIM). For SIM analyses the electron multiplier voltage was raised by 300 V after the elution of lathosterol to increase the sensitivity of detection for lanosterol, dihydrolanosterol, methylsterols, dimethylsterols and plant sterols. The internal standard epicoprostanol was monitored at *m*/*z* 370. In unlabeled experiments, cholesterol precursors and plant sterols were monitored by the following ions: desmosterol (*m*/*z* 456; 441 and 351); Δ8-cholestenol (*m*/*z* 458); lathosterol (*m*/*z* 458); monounsaturated methylsterols (*m*/*z* 472); diunsaturated methylsterols (*m*/*z* 470); monounsaturated dimethylsterols (*m*/*z* 486); diunsaturated dimethylsterols (*m*/*z* 484); triunsaturated dimethylsterols (*m*/*z* 482); lanosterol (*m*/*z* 498 and 393) and dihydrolanosterol (*m*/*z* 500 and 395). Peak integration was performed manually, and sterols were quantified from SIM analyses against the internal standard. Unless stated otherwise, MAS amounts are given as a percentage of the control. When 1-¹³C-acetate was used in the culture medium for ISA, up to 10 ions (M+0- M+10) were monitored for the TMS derivatives of lathosterol (*m*/*z* 458-468), T-MAS (*m*/*z* 484-494) and FF-MAS (*m*/*z* 482-492).

### Δ24-reductase assay

HepG2 cells were incubated for 12 h with 10 µM simvastatin (a kind gift from M. Stapff (MSD, Germany)) to deplete cholesterol precursors. Thereafter, the medium was changed to one containing 10 µM simvastatin, 10 µM miconazole (an inhibitor of 14α-demethylation) and 6 µg/dish lanosterol. After 12 h incubation, neutral sterols were extracted as described above, and the samples were monitored for lanosterol (*m*/*z* 498 (M⁺) and *m*/*z* 393 (M-TMSOH-CH₃)) and dihydrolanosterol (*m*/*z* 500 (M⁺) and *m*/*z* 395 (M-TMSOH-CH₃)) by GC/MS. Dihydrolanosterol was quantified by comparing the ratio of SIM abundances at *m*/*z* 395 and 370 (epicoprostanol) against a standard curve constructed from SIM results on mixtures of authentic dihydrolanosterol and epicoprostanol. Desmosterol was quantified analogously by SIM.

### Isotopomer Spectral Analysis (ISA)

ISA was carried out as described previously (25) by incubating cells with 1-13C-acetate. The isotopomer distribution in sterols synthesized from 1-¹³C-acetate provided the fractional synthesis rate g(t) of the product and the enrichment D of the labeled precursor pool. These parameters were calculated by nonlinear regression analysis as described previously (25,26). Because here we used the molecular ions containing the TMS group for the ISA calculations, the ISA program was modified to correct for the additional C, H and Si atoms from the derivatization reagent. Statistical differences were assessed by Student's t-test and considered significant for *P*<0.05.

### REFERENCES

1. Byskov, A.G., Andersen, C.Y., Nordholm, L., Thogersen, H., Xia, G., Wassmann, O., Andersen, J.V., Guddal, E., and Roed, T. (1995) Chemical structure of sterols that activate oocyte meiosis. *Nature* 374, 559-562
2. Byskov, A.G., and Saxen, L. (1976) Induction of meiosis in fetal mouse testis *in vitro. Dev. Biol.* 52, 193-200
3. Westergaard, L., Byskov, A.G., Andersen, C.Y., Grinsted, J., and McNatty, K.P. (1984) Is resumption of meiosis in the human preovulatory oocyte triggered by a meiosis-inducing substance (MIS) in the follicular fluid? *Fertil. Steril.* 41, 377-384
4. Grondahl, C., Ottesen, J.L., Lessl, M., Faarup, P., Murray, A., Gronvald, F.C., Hegele-Hartung, C., and Ahnfelt-Ronne, I. (1998) Meiosis-activating sterol promotes resumption of meiosis in mouse oocytes cultured in vitro in contrast to related oxysterols. *Biol Reprod* 58, 1297-1302
5. Ruan, B., Watanabe, S., Eppig, J.J., Kwoh, C., Dzidic, N., Pang, J., Wilson, W.K., and Schroepfer, G.J. (1998) Sterols affecting meiosis: novel chemical syntheses and the biological activity and spectral properties of the synthetic sterols. *J. Lipid Res.* 39, 2005-2020
6. Janowski, B.A., Willy, P.J., Devi, T.R., Falck, J.R., and Mangelsdorf, D.J. (1996) An oxysterol signalling pathway mediated by the nuclear receptor LXRα. *Nature* 383, 728-731
7. Forman, B.M., Ruan, B., Chen, J., Schroepfer, G.J., and Evans, R.M. (1997) The orphan nuclear receptor LXRα is positively and negatively regulated by distinct products of mevalonate metabolism. *Proc. Natl. Acad. Sci. U. S. A.* 94, 10588-10593
8. Yoshida, Y., Yamashita, C., Noshiro, M., Fukuda, M., and Aoyama, Y. (1996) Sterol 14-demethylase P450 activity expressed in rat gonads: contribution to the formation of mammalian meiosis-activating sterol. *Biochem. Biophys. Res. Commun.* 223, 534-538
9. Stromstedt, M., Waterman, M.R., Haugen, T.B., Tasken, K., Parvinen, M., and Rozman, D. (1998) Elevated expression of lanosterol 14α-demethylase (CYP51) and the synthesis of oocyte meiosis-activating sterols in postmeiotic germ cells of male rats. *Endocrinology* 139, 2314-2321
10. Byskov, A.G., Baltsen, M., and Andersen, C.Y. (1998) Meiosis-activating sterols: background, discovery, and possible use. *J. Mol. Med.* 76, 818-823
11. Byskov, A.G., Andersen, C.Y., Leonardsen, L., and Baltsen, M. (1999) Meiosis-activating sterols (MAS) and fertility in mammals and man. *J*. *Exp. Zool.* 285, 237-242
12. Grondahl, C., Ottesen, J.L., Lessl, M., Faarup, P., Murray, A., Gronvald, F.C., Hegele-Hartung, C., and Ahnfelt-Ronne, I. (1998) Meiosis-activating sterol promotes resumption of meiosis in mouse oocytes cultured in vitro in contrast to related oxysterols. *Biol. Reprod.* 58, 1297-1302
13. Lin, D.S., Connor, W.E., Wolf, D.P., Neuringer, M., and Hachey, D.L. (1993) Unique lipids of primate spermatozoa: desmosterol and docosahexaenoic acid. *J. Lipid Res.* 34, 491-499
14. Connor, W.E., Lin, D.S., and Neuringer, M. (1997) Biochemical markers for puberty in the monkey testis: desmosterol and docosahexaenoic acid. *J. Clin. Endocrinol. Metab.* 82, 1911-1916
15. Kallio, M.J., Siimes, M.A., Perheentupa, J., Salmenpera, L., and Miettinen, T.A. (1989) Cholesterol and its precursors in human milk during prolonged exclusive breast-feeding. *Am. J. Clin. Nutr.* 50, 782-785
16. Kritchevsky, D., Tepper, S.A., DiTullio, N.W., and Holmes, W.L. (1965) Desmosterol in developing rat brain. *J. Am. Oil Chem. Soc.* 42, 1024-1028
17. Channing, C.P., Hillensjo, T., and Schaerf, F.W. (1978) Hormonal control of oocyte meiosis, ovulation and luteinization in mammals. *Clin. Endocrinol. Metab.* 7, 601-624
18. Schuetz, A.W. (1977) Induction of oocytic maturation and differentiation: mode of progesterone action. *Ann. N. Y. Acad. Sci.* 286, 408-420
19. Panini, S.R., Gupta, A., Sexton, R.C., Parish, E.J., and Rudney, H. (1987) Regulation of sterol biosynthesis and of 3-hydroxy-3-methylglutaryl-coenzyme A reductase activity in cultured cells by progesterone. *J. Biol. Chem.* 262, 14435-14440
20. Haksar, A., Romanoff, E.B., Hagino, N., and Pincus, G. (1967) In vitro inhibition of cholesterol synthesis by pregnenolone in bovine corpus luteum. *Steroids* 9*,* 405-414
21. Metherall, J.E., Li, H., and Waugh, K. (1996) Role of multidrug resistance P-glycoproteins in cholesterol biosynthesis. *J*. *Biol. Chem.* 271, 2634-2640
22. Metherall, J.E., Waugh, K., and Li, H. (1996) Progesterone inhibits cholesterol biosynthesis in cultured cells. Accumulation of cholesterol precursors. *J. Biol. Chem.* 271, 2627-2633
23. Field, F.J., Born, E., Murthy, S., and Mathur, S.N. (1998) Transport of cholesterol from the endoplasmic reticulum to the plasma membrane is constitutive in CaCo-2 cells and differs from the transport of plasma membrane cholesterol to the endoplasmic reticulum. *J*. *Lipid Res.* 39, 333-343
24. Leinonen, P., Ruokonen, A., Kontturi, M., and Vihko, R. (1981) Effects of estrogen treatment on human testicular unconjugated steroid and steroid sulfate production in vivo. *J. Clin. Endocrinol. Metab.* 53, 569-573
25. Kelleher, J.K., Kharroubi, A.T., Aldaghlas, T.A., Shambat, I.B., Kennedy, K.A., Holleran, A.L., and Masterson, T.M. (1994) Isotopomer spectral analysis of cholesterol synthesis: applications in human hepatoma cells. *Am. J. Physiol.* 266, E384-95
26. Holleran, A.L., Lindenthal, B., Aldaghlas, T.A., and Kelleher, J.K. (1998) Effect of tamoxifen on cholesterol synthesis in HepG2 cells and cultured rat hepatocytes. *Metabolism* 47, 1504-1513
27. Hashimoto, F., and Hayashi, H. (1991) Identification of intermediates after inhibition of cholesterol synthesis by aminotriazole treatment in vivo. *Biochim. Biophys. Acta* 1086, 115-124
28. Leinonen, P., Hammond, G.L., and Vihko, R. (1980) Testosterone and some of its precursors and metabolites in the human epididymis. *J. Clin. Endocrinol. Metab.* 51, 423-428
29. Enien, W.M., el-Sahwy, S., Harris, C.P., Seif, M.W., and Elstein, M. (1995) Human chorionic gonadotrophin and steroid concentrations in follicular fluid: the relationship to oocyte maturity and fertilization rates in stimulated and natural in-vitro fertilization cycles. *Hum. Reprod.* 10, 2840-2844
30. Seibel, M.M., Smith, D.M., Levesque, L., Borten, M., and Taymor, M.L. (1982) The temporal relationship between the luteinizing hormone surge and human oocyte maturation. *Am. J. Obstet. Gynecol.* 142, 568-572
31. Lange, Y., Schmit, V.M., and Schreiber, J.R. (1988) Localization and movement of newly synthesized cholesterol in rat ovarian granulosa cells. *Endocrinology* 123, 81-86
32. Lange, Y. (1994) Cholesterol movement from plasma membrane to rough endoplasmic reticulum. Inhibition by progesterone. *J. Biol. Chem.* 269, 3411-3414
33. Lange, Y., and Steck, T.L. (1994) Cholesterol homeostasis. Modulation by amphiphiles. *J. Biol. Chem.* 269, 29371-29374
34. Field, F.J., Born, E., Chen, H., Murthy, S., and Mathur, S.N. (1995) Esterification of plasma membrane cholesterol and triacylglycerol-rich lipoprotein secretion in CaCo-2 cells: possible role of p-glycoprotein. *J. Lipid Res.* 36, 1533-1543
35. Liscum, L., and Underwood, K.W. (1995) Intracellular cholesterol transport and compartmentation. *J. Biol. Chem.* 270, 15443-15466
36. Gerst, N., Ruan, B., Pang, J., Wilson, W.K., and Schroepfer, G.J., Jr. (1997) An updated look at the analysis of C₂₇ sterols by gas chromatography and mass spectrometry. *J. Lipid Res.* 38, 1685-1701
37. Andersen, C.Y., Westergaard, L.G., Sinosich, M.J., and Byskov, A.G. (1992) Human preovulatory follicular fluid: inhibin and free steroids related to optimal follicular maturation in ovarian stimulation regimes and possible function in ovulation. *Hum. Reprod.* 7, 765-769
38. Brailly, S., Gougeon, A., Milgrom, E., Bomsel-Helmreich, O., and Papiernik, E. (1981) Androgens and potent inhibitors in the human ovarian follicle: differences in the evolution of preovulatory, healthy nonovulatory, and atretic follicles. *J. Clin. Endocrinol. Metab.* 53, 128-134
39. McNatty, K.P., Makris, A., DeGrazia, C., Osathanondh, R., and Ryan, K.J. (1979) The production of progesterone, androgens, and estrogens by granulosa cells, thecal tissue, and stromal tissue from human ovaries in vitro. *J. Clin. Endocrinol. Metab.* 49, 687-699
40. Kase, N.G. (1983) The microenvironment of the ovarian follicle. *J. Reprod. Med.* 28, 239-243
41. Zegers-Hochschild, F., Fernandez, E., Mackenna, A., Fabres, C., Altieri, E., and Lopez, T. (1995) The empty follicle syndrome: a pharmaceutical industry syndrome. *Hum. Reprod.* 10, 2262-2265
42. Tsuiki, A., Rose, B.I., and Hung, T.T. (1988) Steroid profiles of follicular fluids from a patient with the empty follicle syndrome. *Fertil. Steril.* 49, 104-107
43. Rodriguez-Rigau, L.J., Weiss, D.B., Smith, K.D., and Steinberger, E. (1978) Suggestion of abnormal testicular steroidogenesis in some oligospermic men. *Acta Endocrinol. Copenh.* 87, 400-412
44. Gondos, B., Byskov, A.G., and Hansen, J.L. (1996) Regulation of the onset of meiosis in the developing testis. *Ann. Clin. Lab. Sci.* 26, 421-425
45. Nikkila, K., Riikonen, S., Lindfors, M., and Miettinen, T.A. (1996) Serum squalene and noncholesterol sterols before and after delivery in normal and cholestatic pregnancy. *J. Lipid Res.* 37, 2687-2695
46. Tomas Kuchta et al., Inhibition of Sterol 4-Demethylation by 6-Amino-2-n-Pentylthiobenzothiazol,
   Antimicrob. Agents Chemother., July 1995, pages 1538 - 1541.
47. Tomas Kuchta et al., Inhibition of ergosterol biosynthesis with the antifungal agent 6-Amino-2-n-Pentylthiobenzothiazol, FEMS Microbiology Letters 150 (1997), 43-47.

**The abbreviations used are:** BSA, bovine serum albumin; DMEM, Dulbecco's modified Eagle's medium; FF-MAS, follicular fluid meiosis-activating sterol; GC/MS, gas chromatography/mass spectrometry; HMG-CoA, 3-hydroxy-3-methylglutaryl coenzyme A; ISA, isotopomer spectral analysis; MAS, meiosis-activating sterols; PBS, phosphate buffered saline; SIM, selective ion monitoring; T-MAS, testicular meiosis-activating sterol; TMS, trimethylsilyl;
UDCA = Ursodeoxycholic acid,
CDCA = Chemodeoxycholic acid,
CA = Cholic acid,
LCA = Lithocholic acid.

### Legends for Illustrations

**Figure 1.** Late steps of cholesterol synthesis. Numbers in brackets refer to chromatographic peaks in subsequent figures. The vertical dotted line crosses the Δ24-reduction steps, which were inhibited by progesterone, pregnenolone and 17α-OH-pregnenolone. The horizontal dotted line crosses the 4α-demethylation steps, which were inhibited by 17α-OH-progesterone.
**Figure 2.** Cholesterol precursor profile in HepG2 cells. Cells were incubated for 12 h with (A) no added compound (control); (B) progesterone; (C) 17α-OH-progesterone and (D) aminotriazole. Sterols were characterized as their TMS derivatives using GC/MS in the full scan mode as follows (the molecular ion is given in parentheses): IS, internal standard (epicoprostanol); 1) desmosterol (*m*/*z* 456); 2) lathosterol (*m*/*z* 458); 3) monounsaturated methylsterol (*m*/*z* 472); 4) monounsaturated methylsterol (*m*/*z* 472); 5) monounsaturated dimethyl sterol (4,4-dimethyl-5α-cholest-8-en-3β-ol) (*m*/*z* 486); 6) lanosterol (*m*/*z* 498) and 7) diunsaturated dimethyl sterol (T-MAS) (*m*/*z* 484). The large peak at 12.5 min represents cholesterol.
**Figure 3.** (A) Influence of potent inhibitors on Δ24-reduction of lanosterol to dihydrolanosterol in HepG2 cells in the presence of miconazole. The conversion of lanosterol to dihydrolanosterol is expressed as the total amount of dihydrolanosterol (µg ± SD) as measured by SIM using a standard curve (n=3 incubations; **P*<0.05 and ^{†}*P*<0.005). (B) Accumulation of desmosterol after incubation of HepG2 cells with either 10 µM potent inhibitors or 1 µM tamoxifen for 12 h, as measured by SIM using a standard curve (n=2 for potent inhibitors and n=3 for tamoxifen).
**Figure 4.** Cholesterol precursor profile in (A) HepG2 cells incubated with progesterone for 24 h and (B) adult rat testis. Mass spectra of (C) peak 7 from the progesterone incubation and (D) peak 7 (T-MAS) from rat testis. Peak 4 in panel A consists of a monunsaturated methyl sterol (*m*/*z* 472) and a diunsaturated methyl sterol (*m*/*z* 470). Both methylsterols showed extensive ¹³C labeling when the cells were incubated with ¹³C-acetate. Therefore, both methyl sterols are likely to be cholesterol precursors and probably contain a 4α-methyl group. Peak 8 represents a triunsaturated dimethyl sterol and was identified as FF-MAS (*m*/*z* 482). See Fig. 2 for definition of other peak labels.
**Figure 5.** (A) Relationship between rat weight and T-MAS levels in rat testis. (B) Inverse correlation between levels of 4,4-dimethyl-5α-cholest-8-en-3β-ol (*m*/*z* 486) and T-MAS (*m*/*z* 484) in the developing rat testis. T-MAS levels were measured against an internal standard of epicoprostanol by GC/MS of their TMS derivatives as a ratio of the relative abundances of distinctive ions (*m*/*z* 484 for T-MAS; *m*/*z* 370 for epicoprostanol). The T-MAS levels were normalized to correspond to 0.1 g of tissue (wet weight). Levels of the dimethyl sterols were quantitated against epicoprostanol analogously.
**Figure 6.** Influence of potent inhibitors on (A) the fractional synthesis of lathosterol (g(t)) and (B) the enrichment D of the acetate precursor pool calculated from lathosterol by isotopomer spectral analysis (ISA). HepG2 cells were incubated with 10 µM potent inhibitors for 12 h in the presence of 1 mM 1-¹³C-acetate, and the ISA results were calculated from the resulting isotope pattern (*m*/*z* 458-468) as described under methods. Data are given as mean ± SD (n=3 incubations; ***P*<0.001; **P*<0.05).
**Figure 7.** Influence of different concentrations of progesterone (A) and of potent inhibitors (10 µM) (B) on the fractional abundance of [M+5]⁺ (*m*/*z* 473) of cholesterol. HepG2 cells were incubated with potent inhibitors for 12 h in the presence of 1 mM 1-¹³C-acetate, and the fractional abundance of [M+5]⁺ was calculated as the abundance of [M+5]⁺ relative to the sum of all isotopomer abundances (M⁺ to [M+9]⁺). Data are given as mean ± SD (n=3 incubations; ***P*<0.001; **P*<0.05).
**Figure 8.** (A) Isotopomer spectral analysis of ¹³C-labeled T-MAS after incubation of HepG2 cells with 10 µM 17α-OH-progesterone for 12 h in the presence of 1 mM 1-¹³C-acetate. Isotopomers of T-MAS were measured from *m*/*z* 484-495. Data are given as mean ± SD (n=3 incubations) of the observed ion abundances compared with abundances predicted by the ISA model. (B and C) Accumulation of labeled T-MAS (B) and FF-MAS (C) in HepG2 cells after incubation with potent inhibitors (10 µM; 12 h) in the presence of 1 mM 1-¹³C-acetate. T-MAS was measured on the ions *m*/*z* 484-495 and FF-MAS on *m*/*z* 482-493, and the results are given as a ratio to the internal standard (mean ± SD; n=3 incubations; ***P*<0.001; **P*<0.05).
**Figure 9.** Influence of progesterone and RU486 on the ratio of dihydrolanosterol to lanosterol (A) and on the accumulation of desmosterol (B) in HepG2 cells. Cells were incubated for 12 h in the presence of progesterone (10 µM), the progesterone receptor blocker RU486 (2 µM) and in combination. Neutral sterols were extracted and measured as described under methods, and desmosterol levels were quantified against an internal standard of epicoprostanol by a standard curve (n=3 incubations).

**TABLE 1.**

| Influence of progestins and drugs on levels of C₂₉-sterols in HepG2 cells and rat testis^{a} | | | | |
|---|---|---|---|---|
| Incubated compound | Conc. (µM) | Trienol FF-MAS (*m*/*z* 482) | Enol T-MAS (*m*/*z* 484) | Dienol (*m*/*z* 486) |
| | | % of control | | |
| *HepG2 cells:* | | | | |
| Progesterone | 10 | 3335 ± 397 | 1000 ± 106 | 26 ± 3 |
| 17α-OH-Progesterone | 10 | 406 ± 52 | 2003 ± 201 | 2384 ± 236 |
| Progesterone + 17α-OH-Progesterone | 10 + 10 | 8834 ± 123 | 3972 ± 106 | 136 ± 1 |
| Pregnenolone | 10 | 213 ± 15 | 308 ± 40 | 4 ± 1 |
| 17α-OH-Pregnenolone | 10 | 242 ± 19 | 780 ± 57 | 52 ± 6 |
| Pregnenolone + 17α-OH-Pregnenolone | 10 + 10 | 347 ± 14 | 904 ± 9 | 5 ± 1 |
| Progesterone | 1 | 156 ± 26 | 192 ± 41 | 77 ± 9 |
| 17α-OH-Progesterone | 1 | 120 ± 14 | 162 ± 15 | 246 ± 26 |
| Progesterone + 17α-OH-Progesterone | 1 + 1 | 414 ± 50 | 933 ± 102 | 270 ± 21 |
| Pregnenolone | 1 | 166 ± 28 | 223 ± 11 | 110 ± 9 |
| Pregnenolone + 17α-OH-Progesterone | 1 + 1 | 167 ± 4 | 321 ± 6 | 206 ± 12 |
| Aminotriazole | 20000 | 229 ± 18 | 610 ± 20 | 725 ± 65 |
| Tamoxifen | 1 | 297 ± 41 | 617 ± 47 | 34 ± 3 |
| Aminotriazole + Tamoxifen | 20000 + 1 | 800 ± 25 | 3443 ± 108 | 278 ± 12 |
| Medroxyprogesterone | 10 | 227 ± 11 | 1181 ± 90 | 509 ± 50 |
| Verapamil | 10 | 498 ± 103 | 416 ± 79 | 18 ± 4 |
| | | | | |

| *Rat Testis:* | | | | |
|---|---|---|---|---|
| Progesterone | 10 | 336 ± 12 | 107 ± 1 | 109 ± 1 |
| 17α-OH-Progesterone | 10 | 171 ± 8 | 201 ± 26 | 199 ± 19 |
| Progesterone + 17α-OH-Progesterone | 10 + 10 | 423 ± 22 | 113 ± 9 | 101 ± 1 |
| Progesterone | 20 | 487 ± 37 | 129 ± 10 | 103 ± 14 |
| 17α-OH-Progesterone | 20 | 240 ± 40 | 225 ± 22 | 223 ± 25 |

| | | | | |
|---|---|---|---|---|
| ^{a} **Footnote to Table 1.**HepG2 cells were incubated with the indicated concentrations of potent inhibitors and drugs (12 h for experiments with 10 µM potent inhibitors and drugs; 24 h for experiments with 1 µM potent inhibitors). Rat testis slices were incubated for 9 h. Sterols were measured as their TMS derivatives (*m*/*z* 482 for trienols, *m*/*z* 484 for dienols, and *m*/*z* 486 for enols) using epicoprostanol (*m*/*z* 370; M-TMSOH) as internal standard (n=3 incubations except for experiments in HepG2 cells with 10 µM compound; n=2). Data were obtained from SIM analyses, which provided much better sensitivity than the GC-MS full scan chromatogram shown in Fig. 2A. Values are given as percent of control ± SD. No absolute data are available. | | | | |

**Table 2:**

| Influence of bile acids (160 µM) on methylsterol-dien, dimethylsterol-dien and dimethylsterol-dien in HepG2 cells^{a}, (n=3). Values are calculated as ratio to the internal standard (epicoprostanol 370 m/z). | | | |
|---|---|---|---|
| bile acid | Methylst.-dien (470 m/z) | T-MAS Dimethylst.-dien (484 m/z) | FF-MAS Dimethylst.-trien (482 m/z) |
| | % of control mean ± SD | | |
| UDCA | **509** ± **19**^{**†**} | **655** ± **84**^{**†**} | **309** ± **51**^{**†**} |
| CDCA | **187** ± **5**^{**†**} | **234** ± **11**^{**†**} | **197** ± **10**^{**†**} |
| DCA | **211** ± **6**^{**†**} | **320** ± **32**^{**†**} | **226** ± **17**^{**†**} |
| CA | **98** ± **2** | **102** ± **18** | **95** ± **21** |
| LCA | **459** ± **29**^{**†**} | **744** ± **155**^{**†**} | **453** ± **88**^{**†**} |

| | | | |
|---|---|---|---|
| ^{a} HepG2 cells were incubated for 12-h with 160 µM of bile acids and results are given as percent of control (mean ± SD for experiments of n=3) differences are calculated by Student's t-test). (* p<0.05; ** p<0.01; ^{†} p<0.001 as calculated by Student's t-test) UDCA = Ursodeoxycholic acid, CDCA = Chemodeoxycholic acid, CA = Cholic acid, LCA = Lithocholic acid. | | | |

**Table 3:**

| Influence of bile acids (160 µM for 9-h) on methylsterol-dien, dimethylsterol-dien and dimethylsterol-dien in liver slices^{a}, (n=2 for UDCA, CDCA, DCA and n=3 for CA and LCA). Values are calculated as ratio to campesterol (472 m/z). | | | |
|---|---|---|---|
| bile acid | Methylst.-dien (470 m/z) | T-MAS Dimethylst.-dien (484 m/z) | FF-MAS Dimethylst.-trien (482 m/z) |
| | % of control mean ± SD | | |
| UDCA | **235** | **633** | **204** |
| CDCA | **181** | **296** | **128** |
| DCA | **144** | **210** | **103** |
| CA | **89** | **56** | **81** |
| LCA | **67** | **172** | **91** |

## Claims

1. A process to increase the concentration of meiosis-activating sterols (MAS) in the cholesterol synthesis wherein potent inhibitors where added.

2. Process of claim 1, wherein the Δ24-reduction or/and 4α-demethylation in the cholesterol synthesis are inhibited.

3. Process of claims 1 wherein the concentration of FF-MAS and/or T-MAS as meiosis-activating sterols (MAS) are increased.

4. Process of claim 1, wherein Progesterone, Pregnenolone, 17α-Hydroxy-pregnenolone, 17α-Hydroxy-progesterone, 4-Androsten-3,17-dione, Testosterone, Medroxyprogesterone, Verapamil, Tamoxifen, Ursodeoxycholic acid, Chenodeoxycholic acid, Deoxycholic acid, Lithocholic acid, Cortisone, Cortisol, 11-Desoxycortisol, 17β-Estradiol, Aldosterone Dehydroepiandrosteron, Norethynodrel, 11-Deoxycorticosterone, Corticosterone, 6-Amino-2-n-pentylthiobenzothiazole or mixtures of them are used as potent inhibitors.

5. The use of potent inhibitors in the cholesterol synthesis to increase the concentration of meiosis-activating sterols (MAS).

6. The use of Progesterone, Pregnesolone, Pregnenolone, 17α-Hydroxy-pregnenolone, 17α-Hydroxy-progesterone, Androsten-3,17-dione, Testosterone, Medroxyprogesterone, Verapamil, Tamoxifen, Ursodeoxycholic acid, Chenodeoxycholic acid, Deoxycholic acid, Lithocholic acid, Cortisone, Cortisol, 11-Desoxycortisol, 17β-Estradiol, Aldosterone Dehydroepiandrosteron, Norethynodrel, 11-Deoxycorticosterone, Corticosterone, 6-Amino-2-n-pentylthiobenzothiazole or mixtures of them to increase the concentration of meiosis-activating sterols (MAS).

7. The use of claims 5 and 6 for the manufacture of a medicament for the control of fertility.

8. A pharmaceutical composition comprising the potent inhibitors of claim 1 in admixture with pharmaceutically acceptable auxiliaries.

9. The use of claim 5 for the microbiology production of meiosis-activating sterols (MAS).
